(19) Europäisches Patentamt
European Patent Office
Office européen des brevets

(11) Veröffentlichungsnummer: **0 069 058**
**B1**

(12) # EUROPÄISCHE PATENTSCHRIFT

(45) Veröffentlichungstag der Patentschrift: **26.02.86**

(51) Int. Cl.⁴: **C 07 D 239/26**, C 07 D 401/04, C 08 F 26/06, C 08 F 226/06, C 07 C 95/02, C 07 D 295/10

(21) Anmeldenummer: **82810241.8**

(22) Anmeldetag: **04.06.82**

(54) **2-Substituierte 5-Vinylpyrimidine, daraus herstellbare Polymere und Verfahren zu deren Herstellung.**

(30) Priorität: **10.06.81 CH 3793/81**

(43) Veröffentlichungstag der Anmeldung: **05.01.83 Patentblatt 83/1**

(45) Bekanntmachung des Hinweises auf die Patenterteilung: **26.02.86 Patentblatt 86/9**

(84) Benannte Vertragsstaaten: **CH DE FR GB IT LI NL**

(56) Entgegenhaltungen:
**EP - A - 0 045 277**

**INORGANIC CHEMISTRY, Band 17, Nr. 9, 1978, Seite 2345-9, WASHINGTON (US), J. CARD et al.: "Poly(styryl) bipyridine: Synthesis and formation of transition-metal complexes and some of their physical, chemical and catalytic properties"**

(73) Patentinhaber: **CIBA-GEIGY AG, Klybeckstrasse 141, CH-4002 Basel (CH)**

(72) Erfinder: **Kvita, Vratislav, Dr., Vogesenstrasse 71, CH-4153 Reinach (CH)**
Erfinder: **Kaschig, Jürgen, Dr., 29 Major Appleby's road, Ardsley New York 10502 (US)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 069 058 B1

LIBER, STOCKHOLM 1986

## Beschreibung

Die Erfindung betrifft neue, in 2-Stellung substituierte 5-Vinylpyr-imidine, daraus herstellbare Polymere, Verfahren zu deren Herstellung sowie die zur Herstellung der 2-substituierten 5-Vinylpyrimidine entwickelten neuen Zwischenprodukte.

Vinylpyrimidine waren bisher nur durch mehrstufige aufwendige Synthesen zugänglich; vgl. z.B. J.Am.Chem.Soc. 76, 1878 (1954), J.Het.Chem. 11, 295 (1974) und Polymer 19, 542 (1978). 5-Vinylpyrimidine eignen sich zur Herstellung von komplexbildenden Polymeren. Die komplexbildende Wirkung von solchen Polymeren ist jedoch relativ gering. Aus der Literatur sind ferner komplexbildende Polymere mit Bipyridinresten sowie Uebergangsmetallkomplexe davon bekannt [vgl. z.B. US-PS 3.810.888; deutsche Offenlegungsschriften 2.037.412 und 2.049.057; Polymer Preprints, Japan, 29, 2, 280 (1980); Inorgan.Chem. 17, No. 9, 2345 (1978); J.Org.Chem. 44, 1095 (1978) und 43, 2958 (1978); J.Am.Chem.Soc. 100:21, 6635 (1978) und Inorg.Chim.Acta 33, L 139 (1979) und 44, L 289 (1980)].

Es wurde nun gefunden, dass man neue 5-Vinylpyrimidine der Formel I

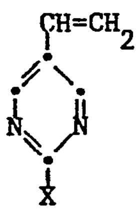

(I) ,

worin

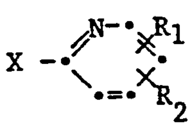

oder

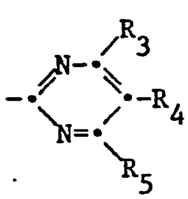

darstellt und $R_1$ bis $R_5$ unabhängig voneinander Wasserstoff, Methyl oder Aechyl darstellen, auf sehr einfache und wirtschaftliche Weise dadurch herstellen kann, dass man 5-Formyl-α-pyron in Gegenwart eines organischen Lösungsmittels mit einem Amin der Formel II

$HN(R_6)(R_7)$ (II)

zu einer Verbindung der Formel III

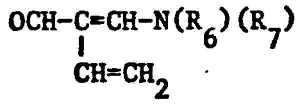

(III)

umsetzt und die Verbindung der Formel III mit einer Verbindung der Formel IV

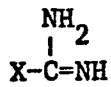

(IV)

zur Reaktion bringt, wobei X die unter Formel I angegebene Bedeutung hat und $R_6$ und $R_7$ unabhängig voneinander unsubstituiertes oder durch eine OH-Gruppe substituiertes geradkettiges oder verzweigtes Alkyl mit bis zu 10 C-Atomen oder zusammen $-C_pH_{2p}$ - oder $-(CH_2)_2$-O-$(CH_2)_2$- und p eine ganze Zahl von 2 bis 22 darstellen.

Bedeutet X eine Gruppe

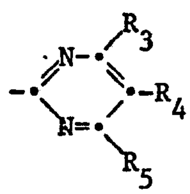

,

so stellen $R_3$, $R_4$ und $R_5$ bevor zugt je Wasserstoff, Methyl oder Aethyl dar. Gemäss einer weiteren-Bevorzugung sind $R_3$ und $R_5$ je Methyl und $R_4$ ist Wasserstoff oder $R_3$ stellt Methyl oder Aethyl dar und $R_4$ und $R_5$ bedeuten je Wasserstoff. Ganz besonders bevorzugt sind Verbindungen der Formel I, worin X die $R_5$ Methyl oder $R_1$ bis $R_5$ je Wasserstoff bedeuten.

Stellen $R_6$ und/oder $R_7$ definitionsgemässe Alkylgruppen dar, so handelt es sich z.B. um die Methyl-, Aethyl-, n-Propyl-, Isopropyl-, n-Butyl-, Isobutyl-, sek.-Butyl-, n-Pentyl-, 2-Methylbutyl-, 2,2-Dimethylpropyl-, n-Hexyl-, n-Heptyl-, 1-Methylhexyl-, 1-Aethylhexyl-, n-Octyl-, n-Decyl-, 2-Hydroxyäthyl-, 2- oder 3-Hydroxypropyl-, 4-Kydroxybutyl-, 6-Hydroxy-hexyl- oder 8-Hydroxyoctylgruppe. Bevorzugt sind geradkettige Alkylgruppen mit 1-8 C-Atomen und Gruppen -(CH) $-CH(CH_3)_2$, worin n Null oder eine ganze Zahl von 1 bis 3 darstellt, besonders Isopropyl, Isobutyl und Isopentyl (2-Methylbutyl). Bilden $R_6$ und $R_7$ zusammen eine Gruppe $-C_pH_{2p}$ -, so kann es sich dabei um geradkettige oder verzweigte Reste handeln. Als geradkettige Reste $-C_pH_{2p}$ - werden solche mit 2-7 und vor allem 2-5 C-Atomen bevorzugt. Bilden $R_6$ und $R_7$ zusammen verzweigte Reste -C $H_2$ -, so kommen insbesondere Reste der Formel V

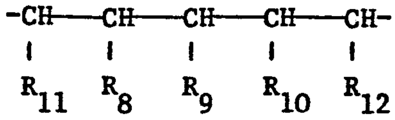

(V)

in Betracht, worin $R_8$ bis $R_{10}$ unabhängig voneinander Wasserstoff oder geradkettiges oder verzweigtes $C_{1-5}$-Alkyl und $R_{11}$ und $R_{12}$ unabhängig voneinander Wasserstoff oder Methyl bedeuten, wobei mindestens eines von $R_8$ bis $R_{12}$ ungleich Wasserstoff ist. Beispiele von geradkettigen oder verzweigten Alkylgruppen $R_8$ bis $R_{10}$ sind: die Methyl-, Aethyl-, n-Propyl-, Isopropyl-, n-Butyl-, sek.-Butyl-, n-Pentyl- und Isopentylgruppe. Vorzugsweise handelt es sich dabei um Aethyl- und besonders um Methylgruppen. Bevorzugt sind Reste der Formel V, worin $R_{11}$ und $R_{12}$ je Methyl und $R_8$ bis $R_{10}$ je Wasserstoff bedeuten.

Als Verbindungen der Formel II verwendet man vorzugsweise solche, worin $R_6$ und $R_7$ unabhängig voneinander geradkettiges $C_{1-8}$-Alkyl, besonders $C_{1-4}$-Alkyl, bedeuten oder zusammen die Reste $-(CH_2)_x-$ mit $x = 2\text{-}5$ oder $-(CH_2)_2\text{-}0\text{-}(CH_2)_2-$ darstellen. Besonders bevorzugt verwendet man Verbindungen der Formel II, worin $R_6$ und $R_7$ unabhängig voneinander Methyl oder Aethyl darstellen oder zusammen eine Gruppe bevorzugt stellen $R_6$ und $R_7$ je Methyl oder Aethyl dar oder bilden zusammen die Gruppe $-(CH_2)_5-$.

Die Konzentration des 5-Formyl-α-pyrons und des Amins der Formel II im organischen Lösungsmittel liegt zweckmässig zwischen 5 und 50 Gew.%, bevorzugt 10-40 Gew.% und insbesondere zwischen 25 und 30 Gew.%. Das 5-Formyl-α-pyron und das Amin der Formel II werden mit Vorteil in äquimolekularen Mengen eingesetzt.

Geeignete organische Lösungsmittel für die Umsetzung des 5-Formyl-α-pyrons mit dem Amin der Formel II sind z.B. cycloaliphatische oder aromatische Kohlenwasserstoffe, wie Cyclohexan, Benzol, Toluol und Xylole; aliphatische oder cyclische Aether, wie Diäthyläther, Dioxan und Tetrahydrofuran; chlorierte aliphatische Kohlenwasserstoffe, wie Methylenchlorid, Chloroform, Tetrachlormethan, Trichloräthylen und Dichloräthan; $C_{1-4}$-Alkylester von niederen aliphatischen Monocarbonsäuren, wie Essigsäuremethyl-, -äthyl- und -n-butylester; Aethylenglykol-dimethyl- und -diäthyläther; Alkanole mit bis zu 6 C-Atomen, wie Methanol, Aethanol, n-Propanol, Isopropanol und Butanol; aliphatische Ketone, wie Aceton; cyclische Amide, wie N-Methylpyrrolidon, N,N-Dialkylamide von niederen aliphatischen Monocarbonsäuren, wie N,N-Dimethylformamid und N,N-Dimethylacetamid; Dialkylsulfoxide, wie Dimethyl- und Diäthylsulfoxid; Hexamethylphosphorsäuretriamid und Sulfolan; Alkylnitrile mit 2-5 C-Atomen, wie Acetonitril, Propionitril und Butyronitril; cyclische Amine, wie Pyridin, Picolin und Lucidin.

Bevorzugte Lösungsmittel sind Benzol, Dioxan, Aethylenglykoldimethylund -diäthyläther, Methanol, Aethanol, Dimethylsulfoxid und Acetonitril. Besonders bevorzugt sind Benzol, Dioxan, Dimethylsulfoxid und vor allem Acetonitril.

Die Reaktionstemperaturen für die Umsetzung des 5-Formyl-α-pyrons mit den Aminen der Formel II können innerhalb weiter Grenzen variieren und liegen zweckmässig zwischen 20 und 100°C. Besonders bevorzugt ist ein Temperaturbereich von 25 bis 40°C. Das 5-Formyl-α-pyron und die Verbindungen der Formel II sind bekannt oder können auf an sich bekannte Weise hergestellt werden.

Die Zwischenprodukte der Formel III die speziell für die Herstellung der erfindungsgemässen Verbindungen der Formel I entwickelt wurden, sind neu und bilden ebenfalls einen Gegenstand der Erfindung. Dabei gilt in Bezug auf bevorzugte Bedeutungen von $R_6$ und $R_7$ in Verbindungen der Formel III das oben Angegebene. Die Verbindungen der Formel III können gewünschtenfalls isoliert werden, z.B. durch Hochvakuumdestillation oder Chromatographie über Kieselgel. Als Laufmittel eignen sich dabei je nach Art der Substituenten $R_6$ und $R_7$ beispielsweise Chloroform oder Gemische von Chloroform und Aceton. Eine Isolierung der Verbindungen der Formel III ist jedoch im allgemeinen nicht erforderlich. Mit Vorteil werden die die Verbindungen der Formel III enthaltenden Reaktionslösungen direkt mit den Amidinen der Formel IV umgesetzt (Eintopfverfahren). Da die Amidine der Formel IV im allgemeinen wenig stabil sind, werden sie zweckmässig vor der Umsetzung auf an sich bekannte Weise mit geeigneten Basen aus ihren Salzen, z.B. den Hydrochloriden, freigesetzt. Als Basen verwendet man mit Vorteil Alkalimetall- oder Erdalkalimetallalkoholate, wie Magnesiumäthylat, Natriumoder Kaliummethylat, Natrium- oder Kalium-tert-butylat. Besonders bevorzugt ist Natriummethylat. Dabei kann man zuvor isolierte Alkoholate in geeigneten Lösungsmitteln, vorzugsweise denselben, wie sie zur Herstellung der Verbindungen der Formel III verwendet werden, einsetzen, oder aber das Alkoholat wird in situ aus den Erdalkali- bzw. Alkalimetallen und überschüssigem Alkohol gebildet. Schliesslich können die Amidine z.B. auch in einem polaren aprotischen Lösungsmittel, wie Dioxan, Tetrahydrofuran, Essigsäureäthylester, Aethylenglykoldimethyloder -diäthyläther, N,N-Dimethylformamid, N,N-Dimethylacetamid, N-Methylpyrrolidon, Dimethylsulfoxid, Hexamethylphosphorsäuretriamid oder Sulfolan, freigesetzt werden, indem man zum Amidinsalz, das in einem dieser Lösungsmittel suspendiert ist, eine äquimolare Menge Natriumhydrid zugibt. Die so erhaltenen Lösungen der Amidine (Amidinbasen) werden dann nach einer bevorzugten Ausführungsform mit den die Verbindungen der Formel III enthaltenden Reaktionslösungen vermischt und zweckmässig

bei Temperaturen zwischen 25 und 90°C, bevorzugt 60 und 80°C, zur Reaktion gebracht. Anschliessend können die Verbindungen der Formel I auf übliche Weise isoliert und gegebenenfalls gereinigt werden, z.B. durch Chromatographieren oder Sublimieren oder eine Kombination dieser Methoden.

Die Verbindungen der Formel I stellen wertvolle Ausgangsmaterialien zur Herstellung von komplexbildenden oder komplexierten Polymeren dar.

Gegenstand der Erfindung sind daher auch neue, gegebenenfalls vernetzte Polymere, die dadurch erhältlich sind, dass man 2 bis 100 Mo1.% einer Verbindung der Formel I und 0 bis 98 Mo1.% einer Verbindung der Formel A)

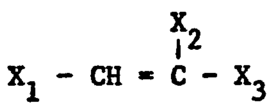 (A),

worin $X_1$ Wasserstoff, $X_2$ Wasserstoff, Chlor oder Methyl und $X_3$ Wasserstoff, Methyl, Chlor, -CN, -COOH, $-CONH_2$, Phenyl, Methylphenyl, Methoxyphenyl, Cyclohexyl, Pyridyl, Imidazolyl, Pyrrolidyl, -COO-Alkyl mit 1-12 C-Atomen im Alkyl, -COO-Phenyl,

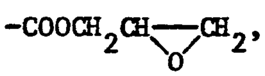

-COO-Alkyl-OH mit 1-4 C-Atomen im Alkyl, -OCO-Alkyl mit 1-4 C-Atomen im Alkyl, -OCO-Phenyl, -CO-Alkyl mit 1-3 C-Atomen im Alkyl, Alkoxy mit 1-20 C-Atomen oder Phenoxy oder $X_2$ Wasserstoff und $X_1$ und $X_3$ zusammen eine Anhydrid-Gruppierung, eine Gruppierung -CO-NR'''-CO- oder je -COOH oder -COOAlkyl mit 1-6 C-Atomen im Alkyl darstellen, wobei R''' geradkettiges oder verzweigtes $C_{1-18}$-Alkyl, Cyclohexyl oder Phenyl bedeutet, das durch $C_{1-6}$-Alkyl, Halogen, Cyano, Nitro und/oder $C_{1-3}$-Alkoxy mono- oder disubstituiert sein kann, in Gegenwart von 0 bis 60 Mo1.% eines mehrfach ungesättigten Vernetzungsmittels polymerisiert und gegebenenfalls so erhaltene komplexbildende Polymere in Polymere überführt, die ganz oder teilweise mit Metallen oder Metallverbindungen komplexiert sind, die keine Alkalimetalle oder Erdalkalimetalle bzw. keine Alkalimetall- oder Erdalkalimetallverbindungen sind.

Dabei kann die Vernetzung auch durch Koordination des Metallatoms, wie Kupfer, Eisen oder Ruthenium, mit weiteren von Verbindungen der Formel I abgeleiteten Strukturelementen erfolgen.

Als Verbindungen der Formel A) werden solche bevorzugt, worin $X_1$ Wasserstoff, $X_2$ Wasserstoff oder Methyl und $X_3$ Phenyl, Pyridyl, -COO-Alkyl-OH mit 2-4 C-Atomen im Alkyl oder -COO-Alkyl mit 1-12 C-Atomen im Alkylteil bedeuten. Ganz besonders bevorzugt ist Styrol.

Geeignete mehrfach ungesättigte Vernetzungsmittel sind z.B. Divinylbenzole, Divinylpyridine, Divinyltoluole, Divinylnaphthaline, Divinylxylole, Divinyläthylbenzole, Divinylsulfon, Divinylketon, Divinylsulfid, Divinylsebacat, Trivinylbenzole, Trivinylnaphthaline, Polyvinylanthracene; Aethylenglycoldiacrylat, Aethylenglycoldimethacrylat, Edlylacrylat, Diallylphthalat, Diallylmaleat, Diallylfumarat, Diallylsuccinat, Diallylcarbonat, Diallylmalonat, Diallyloxalat, Diallyladipat, Diallylsebacat, Diallyltartrat, Diallylsilicat, Triallyltricarballylat, Triallylaconitat, Triallylcitrat, Triallylphosphat, N,N'-Methylendiacrylamid, N,N'-Methylendimethylacrylamid, N,N'-Aethylendiacrylamid, Polyallyl- und Polyvinyläther von Aethylenglycol, Propantriol, Pentaerythritol, Resorcinol und den Monothio- oder Dithioderivaten von Aethylenglycol. Das Vernetzungsmittel wird vorzugsweise in einer Menge von 1 bis 30 Mo1.% eingesetzt. Bevorzugte Vernetzungsmittel sind Divinylpyridin und vor allem Divinylbenzol. Durch geeignete Wahl der Comonomeren und/oder Vernetzungsmittel kann der Quellungsgrad der Polymeren den gewünschten spezifischen Anwendungen angepasst werden.

Bevorzugt sind lineare Polymere mit einem durchschnittlichen Molekulargewicht von 1000 bis 5 000 000, die aus 3 bis 100 Mo1.% wiederkehrenden Strukturelementen der Formel B)

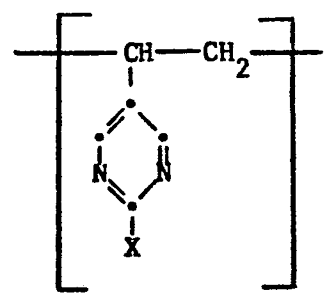 (B)

und/oder Komplexen derartiger Strukturelemente mit Metallen oder Metallverbindungen, die keine Alkalimetalle oder Erdalkalimetalle bzw. keine Alkalimetall- oder Erdalkalimetallverbindungen sind, und 0 bis 97 Mol.% wiederkehrenden Strukturelementen der Formel C)

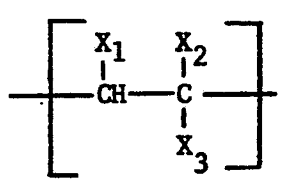 (C)

und $X_3$ die unter Formel A) angegebene Bedeutung haben, insbesondere derartige Polymere mit einem durchschnittlichen Molekulsrgewicht von 2000 bis 3 000 000, die aus 3 bis 100 Mo1.% wiederkehrenden

Strukturelementen der Formel B) und/oder definitionsgemässen Komplexen derartiger Strukturelemente der Formel B) und aus 0 bis 97 Mo1.% wiederkehrenden Strukturelementen der Formel C) bestehen, wobei $R_1$ bis $R_5$, $X_1$, $X_2$ und $X_3$ die im Vorangehenden angegebenen bevorzugten Bedeutungen haben und 5 bis 100 Prozent der Strukturelemente der Formel B) mit Metallen oder Metallverbindungen komplexiert sind, die keine Alkalimetalle oder Erdalkalimetalle bzw. keine Alkalimetall- oder Erdalkalimetallverbindungen sind. Ganz besonders bevorzugt sind derartige lineare Polymere mit einem durchschnittlichen Molekulargewicht von 2000 bis 1 500 000 und/oder deren definitionsgemässe Komplexe, die nur aus Strukturelementen der Formel B) und/oder definitionsgemässen Komplexen davon bestehen, worin X die unter Formel I angegebene Bedeutung hat, $R_1$, $R_2$ und $R_4$ Wasserstoff und $R_3$ und $R_5$ Methyl oder $R_1$ bis $R_5$ je Wasserstoff darstellen und 5 bis 100 Prozent der Strukturelemente der Formel B) definitionsgemäss komplexiert sind.

Die obigen linearen Polymeren können dadurch erhalten werden, dass man 3 bis 100 Mo1.% einer Verbindung der Formel 1 mit 0 bis 97 Mo1.% einer Verbindung der Formel A) polymerisiert und gegebenenfalls anschliessend so erhaltene komplexbildende Polymere in Polymere überführt, die ganz oder teilweise mit Metallen oder Metallverbindungen komplexiert sind, die keine Alkalimetalle oder Erdalkalimetalle bzw. keine Alkalimetalloder Erdalkalimetallverbindungen sind.

Die Polymerisation von Verbindungen der Formel I sowie deren Copolymerisation mit Verbindungen der Formel A), gegebenenfalls in Gegenwart von mehrfach ungesättigten Vernetzungsmitteln, kann auf an sich bekannte Weise vorgenommen werden, z.B. in Gegenwart üblicher anionischer Initiatoren. Bevorzugt ist die radikalische Polymerisation. Dabei verwendet man zweckmässig etwa 0,01 bis 5 Gew.%, vorzugsweise 0,01 bis 1,5 Gew.-%, bezogen auf das Gesamtgewicht der Monomeren und allfälliger Vernetzungsmittel, an sich bekannter Radikalinitiatoren, wie anorganische oder organische Peroxide oder Azoverbindungen, z.B. Wasserstoffperoxid, Kaliumperoxidisulfat, tert.-Butylhydroperoxid, Di-tert.-butylperoxid, Peressigsäure, Dibenzoylperoxid, Diacylperoxide, Cumolhydroperoxid, tert.-Butylperbenzoat, tert.-Alkylperoxidcarbonate und $\alpha,\alpha'$-Azoisobutyronitril. Die Reaktionstemperaturen für die radikalische Polymerisation liegen im allgemeinen zwischen etwa 30 und 100°C. Die radikalische Polymerisation kann aber auch in der Kälte durchgeführt werden, wozu man auch Redoxsysteme in den oben genannten Konzentrationen verwenden kann, z.B. Gemische aus Peroxiden, wie Wasserstoffperoxid, und einem Reduktionsmittel, wie zweiwertige Eisenionen. Die Polymerisation kann in homogener Phase, z.B. in Substanz oder in Lösung, oder in heterogener Phase, d.h. als Fällungspolymerisation, Emulsions- oder Suspensionspolymerisation, erfolgen. Bevorzugt ist die Polymerisation in Lösung. Geeignete Lösungsmittel sind z.B. Toluol, N,N-Dimethylformamid, N,N-Dimethylacetamid und Acetonitril.

Als Metalle zur Bildung von Komplexen mit definitionsgemässen Polymeren kommen z.B. solche der Hauptgruppen IIIa und IVa sowie der Nebengruppen IVb Vb VIb VIIb VIII Ib und IIb des Periodischen Systems in Betracht Geeignete Metallverbindungen zur Herstellung definitionsgemässer Komplexe bzw. in definitionsgemässen Komplexen sind vor allem neutrale oder ionische Metallverbindungen der vorgenannten Haupt- und Nebengruppen des Feriodischen Systems, z.B. Salze oder Säuren, wobei bei den Salzen das Metall sowohl im Anion als auch im Kation vorliegen kann. Das Metallatom des Komplexes bzw. der zu dessen Herstellung verwendeten Metallverbindungen kann gegebenenfalls zusätzlich noch andere koordinative, kovalente oder ionische Bindungen aufweisen, die es mit anderen Ionen, Atomen, Molekülen oder Molekülteilen, wie Strukturelementen eines Polymeren, z.B. einer oder mehreren weiteren Verbindungen der Formel I bzw. davon abgeleiteten Polymerstrukturelementen oder einem 2,2'-Bipyridinrest, verbinden.

Als Salze kommen sowohl Salze mit anorganischen als auch organischen Säuren in Betracht, wie Halogenide, besonders Chloride, Nitrate, Sulfate, Phosphate, Perchlorate und Carboxylate, wie Formiate, Acetate, Propionate und Stearate; ferner Salze, die ein komplexes Anion oder Kation aufweisen, z.B. Oxoderivate von Titan, Vanadium, Zirkon, Molybdän, Hafnium, Niob, Tantal, Wolfram und Uran; anionische Metallkomplexe von Halogenid-, Cyanid-, Thiocyanat-, Thiosulfat- und Orthophosphatanionen, wie Tetrachloroplatinat, Tetrachloropalladat oder Hexathiocyanatochromat.

Als Beispiele derartiger Salze bzw. Komplexe seien genannt: Stannylchlorid, Bleiacetat; Kupfer-(I)- oder -(II)chlorid, -bromid oder -jodid, Kupfer(II)acetat, -nitrat oder -sulfat, Kupfer(I)cyanid, Tetraacetonitrilokupfer(I)perchlorat, Silbernitrat; Zinkchlorid, -cyanid und -rhodanid, Cadmiumchlorid, -cyanid und -rhodanid, Quecksilberjodid oder -cyanid; Zirkoniumtetrachlorid; Vanadium(III)chlorid, Vanadiumoxidsulfat, Ammoniummetavanadat, Niob(V)chlorid, Tantal(V)chlorid, Urantetrachlorid oder -bromid, Uranylnitrat und -acetat; Chromcarbonyl, Chrom(III)chlorid, Hexathiocyanatochromat, Molybdänoxitrichlorid, Molybdäncarbonyl, Wolframoxitrichlorid, Wolframcarbonyl; Mangan(II)chlorid und -jodid; Eisen(III)nitrat, -phosphat, -sulfat oder -acetat, Eisen(II)- oder -(III)chlorid, Ruthenium(III)chlorid, Kaliumpentachlorohydroxyruthenat(IV), Dichloro-bis-(2,2'-bipyridin)ruthenium(II), Kobalt(II)chlorid, Kobalt(II)acetat, -nitrat oder -sulfat,

Rhodium(II)acetat, Rhodium(III)chlorid, Kaliumrhodiumchlorid, Nickel(II)acetat, Nickel(II)bromid oder -chlorid, Nickel(II)sulfat, Palladium(II)chlorid oder -jodid, Palladium(IV)chlorid, Palladiumacetat, Palladiumnitrat, Kaliumtetrachloropalladat, Kaliumtetrachloroplatinat, Kaliumhexachloroplatinat.

Bevorzugt sind Komplexe mit Metallen und Metallverbindungen der Nebengruppen Ib, IIb, IVb, Vb, VIb, VIIb und VIII, besonders Metallen und Metallverbindungen der Nebengruppen Ib und VIII. Ganz besonders bevorzugt sind definitionsgemässe Polymere, die Eisen, Ruthenium, Palladium, Rhodium, Kobalt, Nickel, Platin oder Kupfer, vor allem Ruthenium Palladium, Kobalt, Platin oder Kupfer und ganz besonders Palladium oder Kobalt als komplexiertes Zentralatom enthalten.

Als Säuren kommen z.B. Säuren, die den zuvor genannten Salzen mit einem komplexen Anion entsprechen, in Betracht, wie $H_2PtCl_6$ oder $H_2PdCl_4$.

Werden für die Komplexierung der Polymeren Metallkomplexe eingesetzt, so werden Metallkomplexe mit mindestens zwei leicht ersetzbaren Liganden, welche Ligandenaustausch eingehen können, bevorzugt. Die Wertigkeit des komplexierten Metalls wird durch die Art der für die Komplexherstellung verwendeten Metallverbindungen oder durch eine Oxidationsbzw. Reduktionsreaktion während oder nach der Komplexbildung bestimmt.

Die Ueberführung in definitionsgemässe komplexierte Polymere erfolgt in an sich bekannter Weise, z.B. indem man die Polymeren mit einer Lösung oder Suspension einer geeigneten Metallverbindung in Kontakt bringt. Geeignete Reaktionsmedien sind z.B. Wasser, N,N-Dimethylformamid, N,N-Dimethylacetamid, Acetonitril, Dioxan und Tetrahydrofuran.

Als Metallverbindungen können z.B. Metallsalze oder Säuren der vorerwähnten Art, vor allem Halogenide oder Carboxylate oder aber Metall-Komplexe mit mindestens zwei ersetzbaren koordinierten Liganden, die Ligandenaustausch bewirken können, verwendet werden, wie Tetraacetonitrilo-Kupfer(I)perchlorat, Dichloro-bis-(2,2'-bipyridin)ruthenium-(II) und Kaliumtetrachloroplatinat.

Erfindungsgemäss hergestellte, ganz oder teilweise komplexierte Polymere können als Katalysatoren, z.B. als Hydrierungskatalysatoren, wie zur Hydrierung von Alkenen oder Alkinen, als Isomerisierungskatalysatoren oder als Katalysatoren für die Acetoxylierung von Benzol eingesetzt werden.

Erfindungsgemässe, mindestens teilweise mit Palladium oder einer Palladiumverbindung, besonders Palladiumacetat, komplexierte Polymere eignen sich mit guter Effizienz vor allem als Katalysatoren für Umvinylierungsreaktionen. Ihre Wirkung ist mit derjenigen homogener Katalysatoren, wie sie im allgemeinen für Umvinylierungsreaktionen verwendet werden, vergleichbar. Gegenüber vorbekannten Umvinylierungskatalysatoren, wie Kaliumtetrachloropalladat, haben sie jedoch den Vorteil, dass sie ohne Wiederaufbereitung direkt für weitere Reaktionen verwendet werden können.

Erfindungsgemässe nicht-komplexierte Polymere können zur Herstellung von entsprechenden komplexierten Polymeren verwendet werden. Derartige komplexbildende Polymere finden aber auch Anwendung als Metallionen-Fänger, z.B.

- für die Extraktion von Edelmetallen, Seltenen Erden und radioaktiven Elementen, wie Uran, aus ihren Erzen oder Mineralien,
- für die Trennung von radioaktivem Cäsium von anderen Metallen, oder die Trennung verschiedener Metalle (mit der Ausnahme von Alkalimetallen und Erdalkalimetallen),
- für die Gewinnung von Chromsalzen aus Gerbereiabwässern,
- für die Entmineralisierung von organischen Lösungsmitteln ohne Einbringen von Fremdionen zur Herstellung von dielektrischen Flüssigkeiten,
- für die Reinigung von Industrieabwässern zur Entfernung von unerwünschten Metallionen.

Nach dem Binden der Metallionen können die erhaltenen Komplexe wieder in komplexbildende Polymere übergeführt werden, z.B. durch Elution mit starken Säuren oder mit komplexbildenden Mitteln, wie Aethylendiamin oder Aethylendiaminotetraessigsäure.

Mit den Verbindungen der Formel I lassen sich lineare Polymere mit praktisch beliebigem durchschnittlichem Molekulargewicht herstellen. Durch Verwendung von geeigneten Comonomeren und/oder Vernetzungsmitteln können sogenannte massgeschneiderte Polymere hergestellt werden, d.h. Polymere mit den spezifischen Anwendungen angepasster Zusammensetzung und Zahl von komplexbildenden oder komplexierten Pyrimidineinheiten. Die mit den Verbindungen der Formel I herstellbaren, gegebenenfalls vernetzten Polymeren zeichnen sich zudem durch eine hohe Stabilität gegen thermischen oder chemischen Abbau aus.

Die folgenden Beispiele dienen der Erläuterung der Erfindung.

## A) Herstellungsbeispiele

### Beispiele 1-12: Herstellung von Verbindungen der Formel III

Aequimolekulare Mengen 5-Formyl-α-pyron und Amin der Formel II werden in den in der untenstehenden Tabelle angegebenen Lösungsmitteln gelöst. Die erhaltene Lösung wird gerührt, bis aus der Lösung kein Kohlendioxid mehr entweicht. Die weiteren Reaktionsbedingungen sowie die NMR-Spektren der erhaltenen Verbindungen der Formel III sind in der folgenden Tabelle angegeben. Die so

hergestellten Verbindungen der Formel III können direkt für die weitere Umsetzung verwendet werden.

**Tabelle:** Verbindungen der Formel III

a = Quartett
b = Quartett
c = Quartett
d = Singulett
e = Duplett

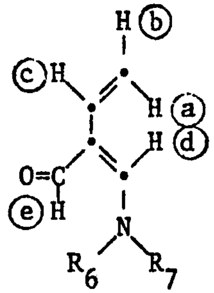

| Bsp. Nr. | $R_6$ = $R_7$ | Lösungsmittel | Zeit (Std.) | T (°C) | NMR-Spektrum a ppm | b ppm | c ppm | d ppm | e ppm | Jab (Hz) | $J_{ce}$ (Hz) | Jac (Hz) | $J_{bc}$ (Hz) |
|---|---|---|---|---|---|---|---|---|---|---|---|---|---|
| 1 | Methyl | $CD_3CN$ *) | 5 | 40 | 5,60 | 5,10 | 6,58 | 6,70 | 8,97 | 3 | 1 | 17 | 11 |
| 2 | Aethyl | Benzol ($d_6$) | 2 | 25 | 6,10 | 5,25 | 6,50 | 6,30 | 9,28 | (Verbindungen Nr. 1-12) | | | |
| 3 | n-Pentyl | $CD_3CN$ | 5 | 25 | 5,60 | 5,10 | 6,40 | 6,65 | 8,95 | | | | |
| 4 | n-Octyl | $CD_3CN$ | 8 | 25 | 5,60 | 5,08 | 6,38 | 6,62 | 8,94 | | | | |
| 5 | Isopropyl | $CD_3CN$ | 7 | 25 | 5,59 | 5,10 | 6,37 | 6,80 | 9,01 | | | | |
| 6 | Isobutyl | $CD_3CN$ | 5 | 25 | 5,50 | 5,20 | 6,48 | 6,72 | 9,00 | | | | |
| 7 | 2-Methylbutyl | $CD_3CN$ | 5 | 25 | 5,60 | 5,10 | 6,40 | 6,64 | 8,84 | | | | |
| 8 | $-CH(CH_3)-(CH_2)_3-CH(CH_3)$ | $CD_3CN$ | 10 | 25 | 5,60 | 5,08 | 6,41 | 6,62 | 8,94 | | | | |
| 9 | $-(CH_2)_3-$ | $CD_3CN$ | 3 | 40 | 5,84 | 5,00 | 6,40 | 6,55 | 8,92 | | | | |
| 10 | $-(CH_2)_4-$ | Dimethylsulfoxid ($d_6$) | 5 | 40 | 5,75 | 4,93 | 6,53 | 6,99 | 8,90 | | | | |
| 11 | $-(CH_2)_5-$ | $CD_3CN$ | 2 | 25 | 5,50 | 5,10 | 6,38 | 6,68 | 8,93 | | | | |
| 12 | $-(CH_2)_2-O-(CH_2)_2$ | $CD_3CN$ | 2 | 25 | 5,47 | 5,14 | 6,38 | 6,70 | 8,96 | | | | |

*) $CD_3CN$ = Trideuteroacetonitril

**Beispiel 13:** 2-Pyrimidyl-5-vinylpyrimidin.

53,32 g (0,43 Mol) 5-Formyl-α-pyron werden in 344 ml Acetonitril gelöst und dann unter Eiskühlung mit 36,98 g (0,43 Mol) Piperidin versetzt. Fast sofort tritt eine starke Entwicklung von Kohlendioxid ein. Nach 2 Stunden Rühren bei Zimmertemperatur (24° C) ist die Reaktion beendet.

Getrennt setzt man aus 67,56 g (0,43 Mol) Pyrimidylamidin-hydrochlorid durch Zugabe von 75,44 g (0,43 Mol) Natriummethylat in 260 ml Methanol die 2-Pyrimidinamidinbase frei. Die Acetonitrillösung von α-Vinyl-β-piperidylacrolein wird mit der methanolischen Lösung der 2-Pyrimidinamidinbase vermischt und 6 Stunden am Rückfluss gekocht. [Den vollständigen Verlauf der Reaktion kann man mittels Dünnschichtchromatographie verfolgen, obwohl das α-Vinyl-β-piperidylacrolein denselben-$R_f$-Wert hat wie das 2-Pyrimidyl-5-vinylpyrimidin (Silicagel, Dünnschichtplatte; Laufmittel Chloroform/Aceton im Vol.-Verhältnis 9:1). Der Fleck des Eduktes färbt sich nach Besprühen mit Ninhydrin und Aufheizen der Platte braun, während der Fleck des Produktes bei derselben Behandlung farblos bleibt.] Anschliessend wird das Reaktionsgemisch filtriert, und das Filtrat wird zur Trockne eingedampft. Das Rohprodukt wird über eine Silicagelsäule (1000 g SiO) chromatographiert (Laufmittel Chloroform/Aceton im Volumenverhältnis 9:1). Das erhaltene Produkt wird bei 130° C/10 $^{-2}$ bar sublimiert. Man erhält 15,8 g (20% d.Th.) 2-Pyrimidyl-5-vinylpyrimidin; Fp. 150° C.

Analyse für $C_{10}H_8N_4$ (Molgewicht 184,2):
berechnet C 65,21% H 4,38% N 30,42%
gefunden C 65,10% H 4,59% N 30,12%

NMR-Spektrum: Vinylgruppe δ = 5,58 ppm (1H), Duplett (J=10Hz), 6,00 ppm (1H, Duplett, J=18Hz), 6,75 ppm (1H, Quartett, J=18Hz und J=10Hz). pyrimidinringe δ= 7 40 ppm (IH Triplett J=5Hz) 9,05 ppm, (2H, Duplett, J=5Hz), 9,05 ppm (2H, Singulett).

**Beispiel 14.** 2-Pyridyl-5-vinylpyrimidin.

10,54 g (0,058 Mol) 5-Formyl-α-pyron werden in 68 ml Acetonitril gelöst und dann unter Eiskühlung mit 7,31 g (0,085 Mol) Piperidin versetzt. Es tritt eine starke Kohlendioxidentwicklung ein. Nach 2 Stunden Rühren bei Zimmertemperatur (24° C) ist die Reaktion beendet. Getrennt setzt man durch Eintragen von 13,34 g (0,085 Mol) 2-Pyridylamidinhydrochlorid in eine Natriummethylatlösung [1,96 g (0,059 Grammatom) Natrium und 68 ml Methanol] die 2-Pyridylamidinbase frei. Die Acetonitrillösung des α-Vinyl-β-piperidylacroleins wird mit der methanolischen Lösung der β-Pyridylamidinbase vermischt und 12 Stunden am Rückfluss gekocht. Anschliessend wird das Reaktionsgemisch abgenutscht, und der pH wird mit Essigsäure auf 4 eingestellt. Die wässrige Lösung wird mit drei 200 ml-Portionen Chloroform extrahiert. Die Extrakte werden zur Trockne eingeengt und der Destillationsrückstand wird über eine Silicagelsäule chromatographiert (250 g Silicagel, Laufmittel Chloroform/Methanol im Vol.-Verhältnis 9:1). Der nach dem Einengen des Laufmittelgemisches erhaltene Rückstand wird heiss mit sechs 50 ml-Portionen Cyclohexan extrahiert. Aus den vereinigten Extrakten kristallisiert das 2-Pyridyl-5-vinylpyrimidin aus. Dieses wird bei 90° C/10 $^{-2}$ bar sublimiert. Man erhält 5,44 g (35% d.Th.) 2-Pyridyl-5-vinylpyrimidin; Fp. 103° C.

Analyse für $C_{11}H_9N_3$ (Molgewicht 183,21):
berechnet C 72,12% H 4,95% S 22,94%
gefunden C 71,86% H 4,99% S 23,00%.

NMR-Spektrum: Vinylgruppe δ = 5,48 ppm (1H, Duplett, J = 11 Hz), 5,91 ppm (1H, Duplett, J = 18 Hz), 6,70 ppm (1H, Quartett, J = 18 Hz und J = 11 Hz); Pyridinring δ= 7,36 ppm (IH, Oktett, J = 5 Hz, J = 8 Hz, J = 1 Hz), 7,82 ppm (IH, Sextett, J = 8 Hz, J = 8 Hz, J = 2 Hz), 8,40 ppm (1H, Quartett, J = 8 Hz, J = 1 Hz), 8,86 ppm (1H, Quartett, J = 5 Hz, J = 2 Hz).

**Beispiel 15.** Analog Beispiel 13 stellt man aus 12 94 g (0,104 Mol)

5-Formyl-α-pyron und 8,84 g oder 10,26 ml (0,104 Mol) Piperidin in 84 ml Acetonitril eine Lösung von α-Vinyl-β-piperidylacrolein her. Getrennt wird aus 19,46 g (0,104 Mol) 4,6-Dimethylpyrimidylamidinhydrochlorid durch Zugabe von 18,31 g (0,104 Mol) Natriummethylat in 70 ml Methanol die entsprechende Base freigesetzt. Die Acetonitrillösung von α-Vinyl-β-piperidylacrolein wird dann mit der methanolischen Suspension der 4,6-Dimethyl-2-pyrimidinamidinbase vermischt und 3 Stunden zum Rückfluss erhitzt. Während dieser Zeit tropft man 6,24 g, d.h. 5,88 ml (0,104 Mol) Eisessig hinzu. Die Suspension wird abgenutscht und mit 20 ml Methanol nachgewaschen. Das Filtrat wird zur Trockne eingeengt, und der Rückstand wird über eine Silicalgelsäule (1000 g $SiO_2$) chromatographiert (Laufmittel Chloroform/Methanol im Volumenverhältnis 19:1). Das erhaltene Produkt (14,1 g = 63,95 % d.Th.) wird mit Diäthyläther verrührt, abgenutscht und bei 80° C/100 bar getrocknet. Man erhält 13,42 g (60,86 % d.Th.) 4,6-Dimethyl-2-pyrimidyl-5-vinylpyrimidin; Fp. 230-232° C. Das Produkt lässt sich bei 160° C/2 x $10^{-3}$ bar gut sublimieren. Der Schmelzpunkt wird dadurch nicht erhöht.

Analyse für $C_{12}H_{12}N_4$ (Molgewicht 212,26):
berechnet C 67,90 % H 5,70 % N 26,40 %
gefunden C 67,93 % H 5,76 % N 26,55 %.

NMR-Spektrum: Vinylgruppe δ= 5,57 ppm (1H, Duplett, J = 10 Hz), 6,01 ppm (1H, Duplett, J = 18 Hz), 6,79 ppm (1H, Quartett, J = 18 Hz und J = 10 Hz).

Pyrimidinringe δ= 7,16 ppm (1H, Singulett),

9,02 ppm (2H, Singulett), 2,69 ppm (6H, Singulett).

**Beispiel 16:** Poly {1-[2-(pyridin-2-yl)-pyrimidin-5-yl]-äthylen}

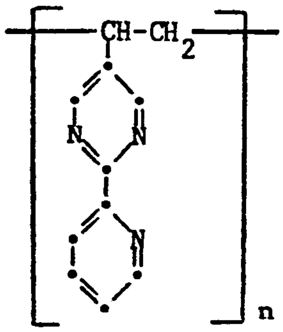

a) Eine Lösung von 1,5 g 2-Pyridyl-5-vinylpyrimidin in 3 ml N,N-Dimethylacetamid wird in einer mit Stickstoff gespülten Ampulle mit 4,03 mg (0,3 Mo1.%) Azobisisobutyronitril (AIBN) gemischt und 16 Stunden unter Luftausschluss auf 70°C erhitzt. Die entstandene hochviskose Mischung wird in Chloroform gelöst. Durch Eingiessen der Lösung in ca. 300 ml Diäthyläther wird ein weisses Pulver erhalten. Ausbeute 1,35 g (90% d.Th.). Grenzviskosität (Chloroform): [η] = 0,49 dl/g, durchschnittliches Molekulargewicht $M_w$ = 141 000.

b) Analog werden 1,5 g 2-Pyridyl-5-vinylpyrimidin in 3 ml Toluol polymerisiert. Es wird ein Fällungspolymerisat erhalten. Ausbeute 1,42 g (95% d.Th.). Grenzviskosität (Chloroform): [η] = 0,453 dL/g, durchschnittliches Molekulargewicht $M_w$ = 162 000.

**Beispiel 17** Poly {1-2- (pyridimin-2-yl) - pyridimin-5-yl]äthylen }

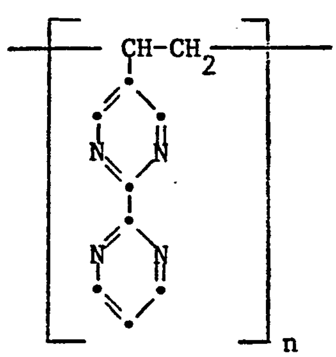

Eine Lösung von 2,2 g 2-Pyrimidyyl5-vinyl-pyrimidin in 7,5 g N,N-Dimethylacetamid wird mit 5,86 mg (0,3 Mo1.%) AIBN gemischt und analog Beispiel 16 zur Polymerisation gcbracht. Das heterogene Reaktionsgemisch wird in Methanol gelöst, und das Produkt wird aus 2,5 ml Toluol gefällt. Ausbeute 1,86 g (85% d.Th.). Grenzviskosität (Methanol) [η] = 0,29 dl/g Durchschnittliches Molekulargewicht $\bar{M}_w$ = 330 000.

**Beispiel 18:**

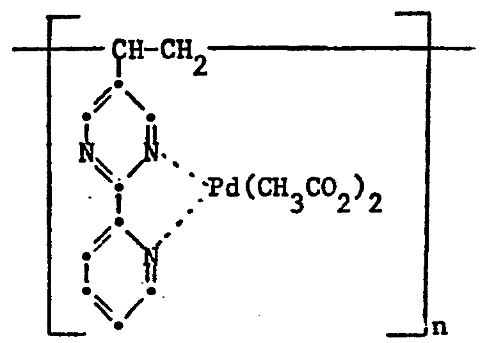

Zu einer Lösung von 275 mg (1,5 mMol) des nach Beispiel 16 erhaltenen Polymeren in 50 ml Tetrahydrofuran (THF) wird unter Stickstoffatmosphäre eine Lösung von 400 mg (1,78 mMol) Palladium(II)acetat in 15 ml THF getropft. Es wird 16 Stunden bei 23°C gerührt. Der gelbbraune Niederschlag wird abgesaugt und mit ca. 500 ml THF gewaschen. Ausbeute 0,53 g (87% d.Th.). Das Produkt hat einen Palladiumgehalt von 24,9 Gew.%. Molverhältnis N:Pd = 3:0,93.

**Beispiel 19:**

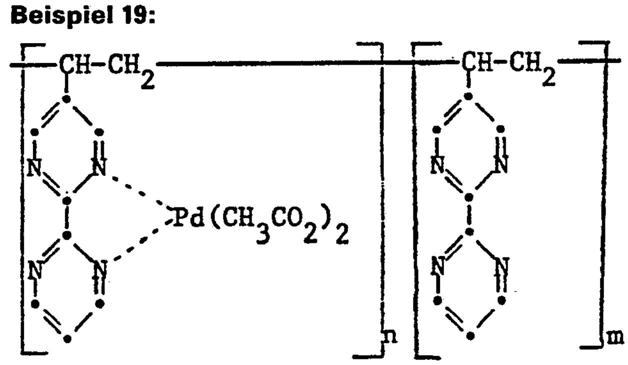

$$\frac{n}{m+n} = 0,192$$

0,4 g (2,17x10 $^{-3}$ Mol) des entsprechend Beispiel 17 erhaltenen Polymeren in 40 ml Wasser wird mit 7 ml THF versetzt. Unter Argonatmosphäre werden bei 23°C 97,4 mg (4,34 x 10 Mol) Palladium(II)acetat, gelöst in 10 ml THF, zugetropft. Nach 18 Stunden Rühren wird im Vakuum auf ca. 1/3 des Volumens eingeengt, und das Wasser wird mittels Dioxan azeotrop abdestilliert. Der gelb-braune Niederschlag wird abfiltriert und bei 50°C/0,67 Pa getrocknet.

Analyse für $(C_{14}H_{14}N_4O_4Pd)_n(C_{10}H_8N_4)_m$.1, 8(n+m) $H_2O$ mit

$$\frac{n}{n+m}$$

= 0,192:

berechnet C 50,31 % H 4,80 % N 21,90 % Pd 8,00 %

gefunden C 50,15 % H 4,28 % N 21,72 % Pd 7,92 %.

**Beispiel 20:** Entsprechend dem in Beispiel 19 beschriebenen Vorgehen

werden 0,4 g des gemäss Beispiel 17 erhaltenen Polymeren mit 1,07 g (4,78 x 10 Mol) Palladium(II)acetat, gelöst in 40 ml THF, umgesetzt. Nach Ablauf der Reaktionszeit wird die Lösung in 800 ml THF eingerührt. Der dunkelbraune Niederschlag wird abfiltriert und getrocknet. Ausbeute 1,16 g. Quellungsgrad (THF): Q = 19. Das Produkt enthält 38,7 Gew.-% Palladium. Molverhältnis N:Pd = 2:1,00.

**Beispiel 21:**

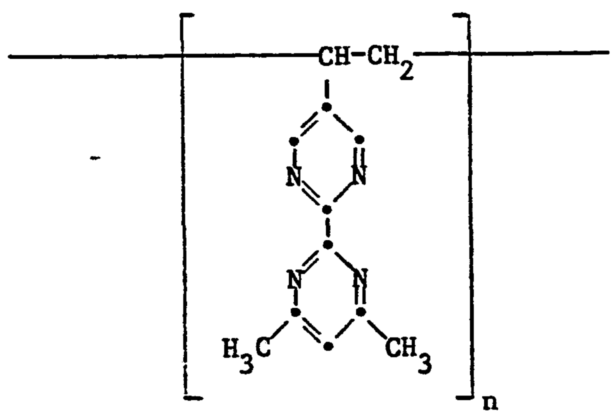

Eine Lösung von 2,53 g (1,19 x 10 Mol) 4,6-Dimethyl-2-pyrimidyl-5-vinylpyrimidin in 70 ml Wasser wird mit 9,6 mg (0,3 Mo1.%) Kaliumperoxidisulfat gemischt und 24 Stunden analog Beispiel 16 zur Polymerisation gebracht. Die entstandene Suspension wird mit ca. 100 ml Wasser verdünnt, und das Produkt wird abgesaugt und mit Wasser gewaschen. Zur Reinigung wird der Filterrückstand in 150 ml Methanol gelöst. Durch Eingiessen der Lösung in ca. 1,5 Liter Wasser wird ein feinteiliger Niederschlag erhalten, welcher durch Zentrifugieren abgetrennt wird. Nach Gefriertrocknung werden 1,93 g (76 % d.Th.) weisses Pulver erhalten. Grenzviskosität (Chloroform): [η] = 1,80 dl/g; durchschnittliches Molekulargewicht

$\overline{M}_w$ = 860 000; Glasumwandlungstemperatur Tg = 85°C.

**Beispiel 22:** Copolymeres aus 2-Pyridyl-5-vinylpyrimidin, Styrol und Divinylbenzol.

Zu 50 ml einer 1%igen Lösung von Polyvinylalkohol in Wasser wird eine Mischung aus 3,66 g (0,02 Mol) 2-Pyridyl-5-vinylpyrimidin, 18,33 g (0,176 Mol) frisch destilliertem Styrol, 0,520 g (0,004 Mol) technischem Divinylbenzol und 0,099g (0,6 mMol) AIBN gegeben. Die Suspension wird unter Stickstoffatmosphäre 16 Stunden bei 70°C sowie 3 8tunden bei 100°C gerührt (400 Umdrehungen/Minute). Das körnige Produkt wird abgesaugt, mit Wasser gewaschen und 2 8tunden in 150 ml siedendem Tetrahydrofuran gequollen. Die Mischung wird anschliessend in 4 Liter Wasser von 75°C eingerührt. Der Niederschlag wird abgesaugt und in einem Soxhlet-Extraktor 16 Stunden mit Tetrahydrofuran gewaschen. Nach dem Trocknen bei 60°C/1,3 Pa werden 15,9 g (70 % d.Th.) weisses körniges Produkt erhalten. Quellfaktor (Tetrahydrofuran): Q = 4,05.

Analyse für $(C_{11}H_9N_3)$ $(C_8H_8)$ $(C_{10}H_{12})$ mit

$$\frac{n}{n+m+o}$$

= 0,145 und

$$\frac{o}{n+m+o}$$

= 0,020:

berechnet C 87,63 % H 7,12 % N 5,25 %

gefunden C 87,12 % H 7,16 % N 4,92 %.

**Beispiel 23:** Copolymeres aus 2-Pyrimidyl-5-vinylpyrimidin, Styrol und Divinylbenzol.

A. Analog Beispiel 22 werden 3,68 g (0,02 Mol) 2-Pyrimidyl-5-vinylpyrimidin, 18,33 g (0,176 Mol) 8tyrol sowie 0,520 g (0,004 Mol) technisches Divinylbenzol copolymerisiert. Zur Aufarbeitung wird das Reaktionsgemisch in 2 Liter Methanol eingerührt, und das feinteilige Produkt wird durch Zentrifugieren abgetrennt. Nach Quellung in 100 ml siedendem Tetrahydrofuran wird das Gel in 4 Liter Wasser von 75°C eingerührt. Der körnige Niederschlag wird in einem Soxhlet-Extraktor 16 Stunden mit Tetrahydrofuran gewaschen.

Nach Trocknen bei 60°C/1,3 Pa werden 10,2 g (45 % d.Th.) weisses Produkt erhalten. Quellungsgrad (Tetrahydrofuran): Q = 3,19.

Analyse für $(C_{10}H_8N_4)$ $(C_8H_8)$ $(C_{10}H_{10})_o$ mit

$$\frac{n}{n+m+o}$$

= 0,02 und

$\frac{o}{n+m+o}$

= 0,02:
berechnet C 91,30 % H 7,64 % N 1,05 %
gefunden C 91,24 % H 7,64 % N 1,04 %.

B. Eine Lösung von 1,84 g (0,01 Mol) 2-Pyrimidyl-5-vinylpyrimidin, 9 17 g (0,088 Mol) 8tyrol und 0,26 g (0,002 Mol) technischem Divinylbenzol in 25 ml N,N-Dimethylacetamid wird mit 25 mg (0 15 mMol) AIBN versetzt und analog Beispiel 16 zur Polymerisation gebracht. Das erhaltene Oel wird wie oben unter A) beschrieben gereinigt. Ausbeute 9,1 g (81 % d.Th.). Quellungsgrad (Tetrahydrofuran): Q = 7,85.

Analyse für $(C_{10}H_8N_4)_n(C_8H_8)$ $(C_{10}H_{10})_o$ mit

$\frac{n}{n+m+o}$

= 0,175 und

$\frac{o}{n+m+o}$

= 0,02:
berechnet C 84,90 % H 6,84 % N 8,26 %
gefunden C 84,20 % H 7,29 % N 8,24 %.

**Beispiel 24:** Kobalt(II)-Komplex von Poly-{1-[2-(pyrimidin-2-yl)-pyrimidin-5-yl]äthylen{.

74 mg (4x10$_{-4}$ Mol) des nach Beispiel 17 erhaltenen Polymeren werden in 13 ml einer Mischung aus Tetrahydrofuran und Methanol (3:10) gelöst. Bei 50°C wird eine Lösung aus 117 mg (4x10 $^{4}$ Mol) Kobalt(II)nitrathexahydrat in 3 ml Methanol zugetropft. Es entsteht ein blauvioletter Niederschlag aus polymerem Kobaltkomplex.

**Beispiel 25:** Kobalt(II)-Komplex von Poly-{I-[2-pyridin-2-yl)-pyrimidin-5-yl]äthylen}

200 mg (1,09 x $10^{-3}$ Mol) des nach Beispiel 16 erhaltenen Polymeren werden in 40 ml einer Mischung aus Tetrahydrofuran und Aethanol (1:1) gelöst. Bei 60°C wird eine Lösung von 318 mg (1,09 x 10 $^{3}$ Mol) Kobalt(II)nitrat-hexahydrat in 5 ml Aethanol zugetropft. Es entsteht ein braunvioletter Niederschlag aus polymerem Kobaltkomplex.

**B) Anwendungsbeispiele**

**Beispiel I:** Eine Mischung aus 44,4 ml (0,48 Mol) Vinylacetat und 9,77 g (0,08 Mol) Benzoesäure wird auf 65°C erwärmt. Es werden 163 mg (3,7 x $10^{-4}$ Aequivalente Pd) des gemäss Beispiel 18 erhaltenen Polymeren (Katalysator) zugegeben: Nach 1, 2, 3, 5, 7, 12, 17, 24 und 30 8tunden werden jeweils 0,6 ml Reaktionsmischung entnommen und der Umsatz gaschromatographisch bestimmt (1-Chlornaphthalin als interner 8tandard). Die Zeit/Umsatz-Kurve zeigt nach 30 8tunden die maximale Ausbeute an Benzoesäurevinylester von 61 % (Gleichgewichtseinstellung).

Es wird vom Katalysator abdekantiert (die überstehende Lösung hat einen Palladiumgehalt von <5 ppm), von entstandener Essigsäure und nicht umgesetzter Benzoesäure durch Ausschütteln mit wässriger Natriumdicarbonat-Lösung befreit und der Benzoesäurevinylester durch fraktionierte Destillation isoliert. Ausbeute 7,11 g (60 % d.Th.); Kp 94°C/1064 Pa.

Der abdekantierte Katalysator wird erneut in der Mischung aus Benzoesäure und Vinylacetat suspendiert und auf 65°C erwärmt. Nach 30 Stunden sind wiederum ca. 7 g Benzoesäurevinylester entstanden.

**Beispiel II:** Entsprechend der in Beispiel I beschriebenen Arbeitsweise werden 460 mg (3,5 x $10^{-4}$ Aequivalente Pd) des gemäss Beispiel 19 erhaltenen Polymeren als Katalysator eingesetzt. Nach 30 Stunden wird eine Ausbeute an Benzoesäurevinylester von 36 % gemessen.

**Beispiel III:** Entsprechend der in Beispiel I beschriebenen Arbeitsweise werden 127 mg (4,7 x $10^{-4}$ Aequivalente Pd) des gemäss Beispiel 20 erhaltenen Polymeren als Katalysator eingesetzt. Nach 30 Stunden wird eine Ausbeute an Benzoesäurevinylester von 37 % gemessen.

**Patentansprüche**

1. Verbindungen der Formel I

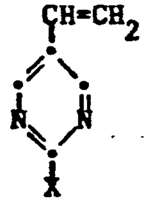

(I) ,

worin X

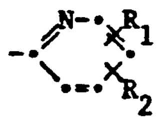

oder

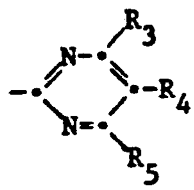

darstellt und $R_1$ bis $R_5$ unabhängig voneinander Wasserstoff, Methyl oder Aethyl darstellen.

2. Verbindungen der Formel I nach Anspruch 1, worin X die angegebene Bedeutung hat, $R_1$, $R_2$ und $R_4$ Wasserstoff und $R_3$ und $R_5$ Methyl oder $R_1$ bis $R_5$ je Wasserstoff darstellen.

3. Verfahren zur Herstellung von Verbindungen der Formel I nach Anspruch 1, dadurch gekennzeichnet, dass man 5-Formyl-α-pyron in Gegenwart eines organischen Lösungsmittels mit einem Amin der Formel II

$HN(R_6)(R_7)$ (II)

zu einer Verbindung der Formel III

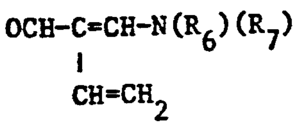

(III)

umsetzt und die Verbindung der Formel III mit einer Verbindung der Formel IV

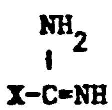

(IV)

zur Reaktion bringt, wobei X die unter Pormel I angegebene Bedeutung hat und $R_6$ und $R_7$ unabhängig voneinander unsubstituiertes oder durch eine OH-Gruppe substituiertes geradkettiges oder verzweigtes Alkyl mit bis zu 10 C-Atomen oder zusammen $-C_pH_{2p}-$ oder $-(CH_2)_2-O-(CH_2)_2-$ und p eine ganze Zahl von 2 bis 22 darstellen.

4. Verbindungen der Formel III

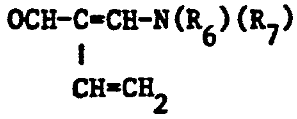

(III) ,

worin $R_6$ und $R_7$ unabhängig voneinsnder unsubstituiertes oder durch eine OH-Gruppe substituiertes geradkettiges oder verzweigtes Alkyl mit bis zu 10 C-Atomen oder zusammen -(C $H_2$) - oder

$-(CH_2)_2-O-(CH_2)_2-$

und p eine ganze Zahl von 2 bis 22 bedeuten.

5. Verbindungen der Formel III nach Anspruch 4, worin $R_6$ und $R_7$ unabhängig voneinander geradkettiges $C_{1-8}$-Alkyl oder zusammen die Reste $-(CH_2)_x-$ mit x = 2-5 oder $-(CH_2)_2-0-(CH_2)_2-$ darstellen.

6. Verbindungen der Formel III nach Anspruch 4, worin $R_6$ und $R_7$ je Methyl oder Aethyl darstellen oder zusammen die Gruppe $-(CH_2)_5-$ bilden.

7. Gegebenenfalls vernetzte Polymere, die dadurch erhältlich sind, dass man 2 bis 100 Mo1.% einer Verbindung der Formel I nach Anspruch 1 und 0 bis 9S Mo1.% einer Verbindung der Formel (A)

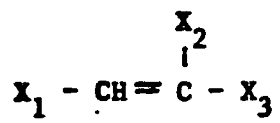

(A) ,

worin $X_1$ Wasserstoff, $X_2$ Wasserstoff Chlor oder Methyl und $X_3$ Wasserstoff, Methyl, Chlor, -CN, -COOH, $-CONH_2$, Phenyl, Methylphenyl, Methoxyphenyl, Cyclohexyl, Pyridyl, Imidazolyl, Pyrrolidyl, -COO-Alkyl mit 1-12 C-Atomen im Alkyl, -COO-Phenyl, $-COOCH_2$

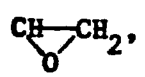

-COO-Alkyl-OH mit 1-4 C-Atomen im Alkyl, -OCO-Alkyl mit 1-4 C-Atomen im Alkyl, -OCO-Phenyl, -CO-Alkyl mit 1-3 C-Atomen im Alkyl, Alkoxy mit 1-20 C-Atomen oder Phenoxy oder $X_2$ Wasserstoff und $X_1$ und $X_3$ zusammen eine Anhydrid-Gruppierung, eine Gruppierung -CO-NR'''-CO- oder je -COOH oder -COO-Alkyl mit 1-6 C-Atomen im Alkyl darstellen, wobei R''' geradkettiges oder verzweigtes $C_{1-18}$-Alkyl, Cyclohexyl oder Phenyl bedeutet, das durch $C_{1-6}$-Alkyl, Halogen, Cyano, Nitro und/oder $C_{1-3}$-Alkoxy monooder disubstituiert sein kann, in Gegenwart von 0 bis 60 Mol.% eines mehrfach ungesättigten Vernetzungsmittels polymerisiert und gegebenenfalls so erhaltene komplexbildende Polymere in Polymere überführt, die ganz oder teilweise mit Metallen oder

Metallverbindungen komplexiert sind, die keine Alkalimetalle oder Erdalkalimetalle bzw. keine Alkalimetall- oder Erdalkalimetallverbindungen sind.

8. Lineare Polymere mit einem durchschnittlichen Molekulargewicht von 1000 bis 5 000 000, die aus 3 bis 100 Mo1.% wiederkehrenden Strukturelementen der Formel (B)

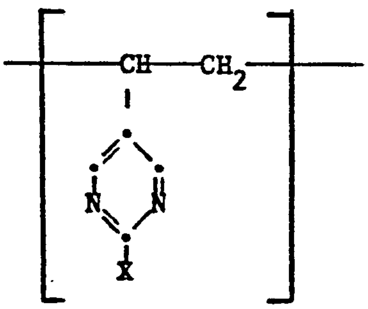

(B)

und/oder Komplexen derartiger Strukturelemente mit Metallen oder Metallverbindungen, die keine Alkalimetalle oder Erdalkalimetalle bzw. keine Alkalimetall- oder Erdalkalimetallverbindungen sind, und 0 bis 97 Mo1.% wiederkehrenden Strukturelementen der Formel (C)

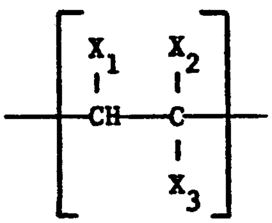

(C)

bestehen, worin X

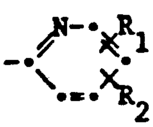

oder

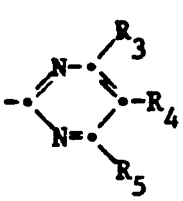

darstellt und $R_1$ bis $R_5$ unabhängig voneinander Wasserstoff, Methyl oder Aethyl, $X_1$ Wasserstoff, $X_2$ Wasserstoff, Chlor oder Methyl und $X_3$ Wasserstoff, Methyl, Chlor, -CN, -COOH, $-CONH_2$, Phenyl, Methylphenyl, Methoxyphenyl, Cyclohexyl, Pyridyl, Imidazolyl, Pyrrolidyl, -COO-Alkyl mit 1-12 C-Atomen im Alkyl, -COO-Phenyl, $-COOCH_2$

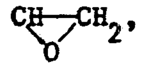

-COO-Alkyl-OH mit 1-4 C-Atomen im Alkyl, -OCO-Alkyl mit 1-4 C-Atomen im Alkyl, -OCO-Phenyl, -CO-Alkyl mit 1-3 C-Atomen im Alkyl, Alkoxy mit 1-20 C-Atomen oder Phenoxy oder $X_2$ Wasserstoff und $X_1$ und $X_3$ zusammen eine Anhydridgruppierung, eine Gruppierung -CO-NR''' -CO- oder je -COOH oder -COO-Alkyl mit 1-6 C-Atomen im Alkyl bedeuten, wobei R''' geradkettiges oder verzweigtes $C_{1-18}$-Alkyl, Cyclohexyl oder Phenyl darstellt, das durch $C_{1-6}$-Alkyl, Halogen, Cyano, Nitro und/oder $C_{1-3}$-Alkoxy mono- oder disubstituiert sein kann.

9. Lineare Polymere mit einem durchschnittlichen Molekulargewicht von 2000 bis 1 500 000 und/oder deren Komplexe nach Anspruch 8, die aus wiederkehrenden Strukturelementen der Formel (B) und/oder definitionsgemässen Komplexen derartiger Strukturelemente der Formel (B) bestehen, worin X die im Anspruch 8 angegebene Bedeutung hat und $R_1$, $R_2$ und $R_4$ Wasserstoff und $R_3$ und $R_5$ Methyl oder $R_1$ bis $R_5$ je Wasserstoff bedeuten, wobei 5 bis 100 Prozent der Strukturelemente der Formel (B) mit Metallen oder Metallverbindungen komplexiert sind, die keine Alkalimetalle oder Erdalkalimetalle bzw. keine Alkalimetalloder Erdalkalimetallverbindungen sind.

10. Komplexe von Polymeren nach Anspruch 7 oder 8, worin das Metall ein Metall der Nebengruppen Ib oder VIII, besonders Eisen, Ruthenium, Palladium, Rhodium, Kobalt, Nickel, Platin oder Kupfer, ist.

11. Komplexe von Polymeren nach Anspruch 7 oder 8, worin das Metall Ruthenium, Palladium, Kobalt, Platin oder Kupfer ist.

12. Verwendung von mindestens teilweise mit Palladium oder einer Palladiumverbindung komplexierten Polymeren nach Anspruch 7 oder 8 als Katalysatoren für Umvinylierungsreaktionen.

**Claims:**

A compound of the formula I

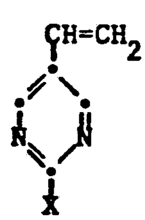

(I) ,

or

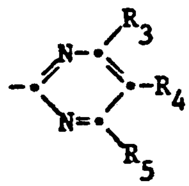

wherein X is

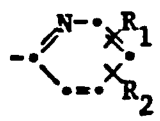

, and each of $R_1$ to $R_5$ independently of the other is hydrogen, methyl or ethyl.

2. A compound of the formula I according to claim 1, wherein X has the given meaning, $R_1$, $R_2$ and $R_4$ are hydrogen and $R_3$ and $R_5$ are methyl, or each of $R_1$ to $R_5$ is hydrogen.

3. A process for the preparation of a compound of the formula I according to claim 1, which process comprises reacting 5-formyl-α-pyrone, in the presence of an organic solvent, with an amine of the formula II

$HN(R_6)(R_7)$ (II)

to give a compound of the formula III

$$\begin{array}{l} OCH-C=CH-N(R_6)(R_7) \\ \quad\ | \\ \quad CH=CH_2 \end{array} \qquad (III),$$

reacting said compound of the formula (III) with a compound of the formula IV

$$\begin{array}{l} \ \ NH_2 \\ \ \ | \\ X-C=NH \end{array} \qquad (IV)$$

in which formulae X is as defined for formula I, each of R and $R_7$ independently of the other is straight chain or branched alkyl which contains up to 10 carbon atoms and is unsubstituted or substituted by an OH group, or $R_6$ and $R_7$ together are $-C_pH_{2p}-$ or $-(CH_2)_2$-0-$(CH_2)$ 2, and p is an integer from 2 to 22.

4. A compound of the formula III

$$\begin{array}{l} OCH-C=CH-N(R_6)(R_7) \\ \quad\ | \\ \quad CH=CH_2 \end{array} \qquad (III),$$

wherein each of $R_6$ and $R_7$ independently of the other is straight chain or branched alkyl which contains up to 10 carbon atoms and is unsubstituted or substituted by an OH group, or together are $-C_pH_{2p}-$ or $-(CH_2)_2$-0-$(CH_2)$ -, and is an integer from 2 to 22.

5. A compound of the formula III according to claim 4, wherein each of $R_6$ and $R_7$ independently of the other is straight chain $C_1$-$C_8$alkyl or together are radicals $-(CH_2)_{x'}$ in which x is 2 to 5' or $(CH_2)_2$-O-$(CH_2)_2$-.

6. A compound of the formula III according to claim 4, wherein each of $R_6$ and $R_7$ is methyl or ethyl or together form the $-(CH_2)_5-$ group.

7. An uncrosslinked or crosslinked polymer which is obtainable by polymerising 2 to 100 mole % of a compound of the formula I according to claim 1, and 0 to 98 mole % of a compound of the formula (A)

$$\begin{array}{l} \qquad\qquad\quad X_2 \\ \qquad\qquad\quad | \\ X_1 - CH = C - X_3 \end{array} \qquad (A) ,$$

wherein $X_1$ is hydrogen, $X_2$ is hydrogen, chlorine or met and $X_3$ is hydrogen, methyl, chlorine, -CN, -COOH, $-CONH_2$, phenyl, methylphenyl, methoxyphenyl, cyclohexyl, pyridyl, imidazolyl, pyrrolidyl, -COO-alkyl containing 1 to 12 carbon atoms in the alkyl moiety, -COO-phenyl,

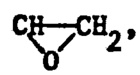

-COO-alkyl-OH containing 1 to 4 carbon atoms in the alkyl moiety, -OCO-alkyl containing 1 to 4 carbon atoms in the alkyl moiety, -OCO-phenyl, -CO-alkyl containing 1 to 3 carbon atoms in the alkyl moiety, alkoxy containing 1 to 20 carbon atoms or phenoxy, or $X_2$ is hydrogen and $X_1$ and $X_3$ together are an anhydride grouping, a -CO-NR'''-CO- grouping or each is -COOH or -COO-alkyl containing 1 to 6 carbon atoms in the alkyl moiety, and R''' is straight chain or branched $C_{1-18}$alkyl, cyclohexyl or phenyl which may be monosubstituted or disubstituted by $C_{1-6}$alkyl, halogen, cyano, nitro and/or $C_{1-3}$alkoxy, in the presence of 0 to 60 mole % of a polyunsaturated crosslinking agent and, if desired, converting a complex-forming polymer so obtained into a polymer which is wholly or partially complexed with a metal other than an alkali metal or alkaline earth metal or with a metal compound other than an alkali metal compound or alkaline earth metal compound.

8. A linear polymer having an average molecular weight of 1000 to 5,000,000 and consisting of 3 to 100 mole % of recurring structural units of the formula (B)

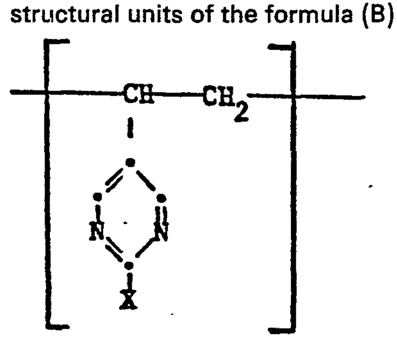

(B)

and/or a complex of such structural units with a metal other than an alkali metal or alkaline earth

metal, or with a metal compound other than an alkali metal compound or alkaline earth metal compound, and 0 to 97 mole % of recurring structural units of the formula C)

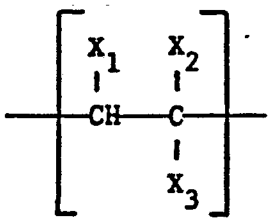

(C)

wherein X is

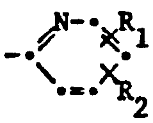

or

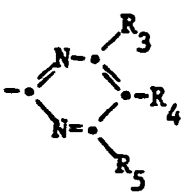

and each of $R_1$ to $R_5$ independently of the other is hydrogen, methyl or ethyl, $X_1$ is hydrogen, $X_2$ is hydrogen, chlorine or methyl, and $X_3$ is hydrogen, methyl, chlorine, -CN, -COOH, $-CONH_2$, phenyl, methylphenyl, methoxyphenyl, cyclohexyl, pyridyl, imidazolyl, pyrrolidyl, -COO-alkyl containing 1 to 12 carbon atoms in the alkyl moiety, -COO-phenyl,

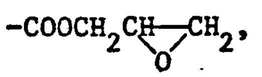

-COO-alkyl-OH containing 1 to 4 carbon atoms in the alkyl moiety, -OCO-alkyl containing 1 to 4 carbon atoms in the alkyl moiety, -OCO-phenyl, -CO-alkyl containing 1 to 3 carbon atoms in the alkyl moiety, alkoxy containing 1 to 20 carbon atoms or phenoxy, or $X_2$ is hydrogen and $X_1$ and $X_3$ together are an anhydride grouping, a -CO-NR''' -CO- grouping or each is -COOH or -COO-alkyl containing 1 to 6 carbon atoms in the alkyl moiety, and R''' is straight chain or branched $C_{1-18}$alkyl, cyclohexyl or phenyl which may be monosubstituted or disubstituted by $C_{1-6}$alkyl, halogen, cyano, nitro and/or $C_{1-3}$alkoxy.

9. A linear polymer having an average molecular weight of 2000 to 1,500,000 and/or a complex thereof according to claim 8, which consists of recurring structural units of the formula (B) and/or of complexes of such structural units of the formula (B), wherein X is as defined in claim 8 and $R_1$, $R_2$ and $R_4$ are hydrogen and $R_3$ and $R_5$ are methyl, or each of $R_1$ to $R_5$ is hydrogen, with 5 to 100 percent of the structural units of the formula (B) being complexed with a metal other than an alkali metal or alkaline earth metal, or with a metal compound other than an alkali metal compound or alkaline earth metal compound.

10. A complex of a polymer according to either claim 7 or 8, wherein the metal is a metal of subgroups Ib or VIII of the Periodic Table, preferably iron, ruthenium, palladium, rhodium, cobalt, nickel, platinum or copper.

11. A complex of a polymer according to either claim 7 or 8, wherein the metal is ruthenium, palladium, cobalt, platinum or copper.

12. Use of a polymer according to either claim 7 or 8, which is at least partially complexed with palladium or a palladium compound, as catalyst for transvinylation reactions.

## Revendications

1.- Composés répondant à la formule I

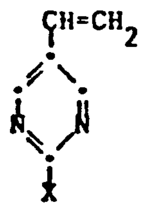

(I)

dans laquelle X représente un radical

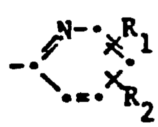

ou

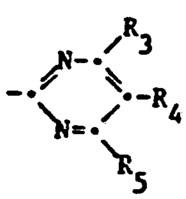

et les symboles $R_1$ à $R_5$ représentent chacun, indépendamment les uns des autres, un atome d'hydrogàne, un radical méthyle ou un radical éthyle.

2.- Composés de formule I selon la revendication 1 dans lesquels X a la signification donnée, $R_1$, $R_2$ et $R_4$ représentent chacun un atome d'hydrogàne, et $R_3$ et $R_5$ chacun un méthyle, ou $R_1$ à $R_5$ représentent chacun un atome d'hydrogène.

3.- Procédé de préparation de composés de formule I selon la revendication 1, procédé caractérisé en ce qu'on fait réagir la formyl-5 α-pyrone, en présence d'un solvant organique, avec une amine de formule II:

$HN(R_6)(R_7)$ (II),

réaction qui donne un composé de formule III:

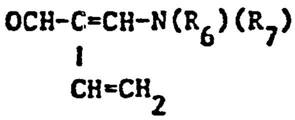

(III),

et on fait réagir le composé de formule III avec un composé de formule IV

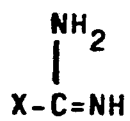

(IV),

formules dans lesquelles X a la signification qui a été donnée au-dessous la formule I tandis que $R_6$ et $R_7$ représentent chacun, indépendamment l'un de l'autre, un radical alkyle linéaire ou ramifié à au plus 10 atomes de carbone, qui ne porte pas de substituant ou qui porte un radical -OH, ou forment endemble un radical $-C_pH_{2p}-$ ou $-(CH_2)_2-O-(CH_2)_2-$, l'indice p désignant un nombre entier de 2 à 22.

4.- Composés répondant à la formule III:

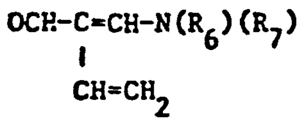

(III)

dans laquelle $R_6$ et $R_7$ représentent chacun, indépendamment l'un de l'autre, un radical alkyle linéaire ou ramifié à au plus 10 atomes de carbone, non substitué ou porteur d'un radical -OH, ou forment ensemble un radical $-C_pH_2$ - ou

$$-(CH_2)_2-O-(CH_2)_2- ,$$

l'indice p désignant un nombre entier de 2 à 22.

5.- Composés de formule III selon la revendication 4 dans lesquels $R_6$ et $R_7$ représentent chacun, indépendamment l'un de l'autre, un radical alkyle linéaire en $C_1$-$C_8$ ou forment ensemble un radical $-(CH_2)_x-$ dans lequel x représente un nombre de 2 à 5, ou un radical $-(CH_2)_2-O-(CH_2)_2-$.

6.- Composés de formule III selon la revendication 4 dans lesquels $R_6$ et $R_7$ représentent chacun un radical méthyle ou éthyle ou forment ensemble un radical $-(CH_2)_5-$.

7.- Polymères éventuellement réticulés, que l'on peut obtenir en polymérisant de 2 à 100 % en moles d'un composé de formule I selon la revendication 1 et de 0 à 98 % en moles d'un composé répondant à la formule A:

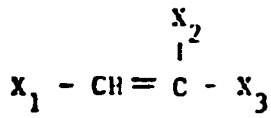

(A)

dans laquelle

$X_1$ représente l'hydrogène,

$X_2$ représente l'hydrogène, le chlore ou un méthyle et

$X_3$ représente l'hydrogène, un méthyle, le chlore, -CN,-COOH,$-CONH_2$, un phényle, un méthylphényle, un méthoxyphényle, un cyclohexyle, un pyridyle, un imidazolyle, un pyrrolidinyle, un alcoxycarbonyle dont la partie alkyle contient de 1 à 12 atomes de carbone, un phénoxycarbonyle,

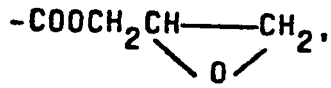

un hydroxyalcoxy carbonyle contenant de 1 à 4 atomes de carbonedans sa partie alkyle, un alkylcarbonyloxy contenant de 1 à 4 atomes de carbone dans sa partie alkyle, un benzoyloxy, un alkylcarbonyle contenant de 1 à 3 atomes de carbone dans sa partie a1ky1e,-un alcoxy en $C_1$-$C_{20}$ ou un phénoxy,

ou

$X_2$ représente l'hydrogène et

$X_1$ et $X_3$ forment ensemble un groupement d'anhydride ou un groupement -CO-NR'''-CO- ou représentent chacun un radical -COOH ou un radical alcoxycarbonyle contenant de 1 à 6 atomes de carbone dans sa partie alkyle, le symbole R''' désignant un alkyle linéaire ou ramifié en $C_1$-$C_{18}$, un cyclohexyle ou un phényle éventuellement porteur d'un ou de deux substituants pris dans l'ensemble constitué par les alkyles en $C_1$-$C_6$, les halogènes, le cyano, le nitro et les alcoxy en $C_1$-$C_3$,

en présence de 0 à 60 % en moles d'un réticulant multi-insaturé, et en transformant éventuellement les polymères complexants ainsi obtenus en polymères complexés, totalement ou partiellement, par des métaux ou des composés de métaux autres que des métaux alcalins ou alcalinoterreux ou des composés de métaux alcalins ou alcalinoterreux.

8.-Polymères linéaires qui ont une masse moléculaire moyenne de 1000 à 5 000 000 et qui sont constitués de 3 à 100 % en moles de motifs de formule B:

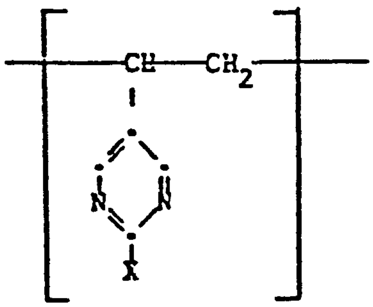

(B)

et/ou de complexes de motifs de ce genre avec des métaux ou des composés métalliques qui ne sont respectivement ni des métaux alcalins ou alcalinoterreux ni des composés de métaux alcalins ou alcalinoterreux, et de 0 à 97 % en moles de motifs de formule C:

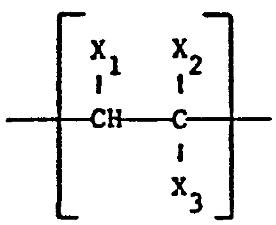

(C) ,

formules dans lesquelles X représente un radical répondant à l'une des formules suivantes:

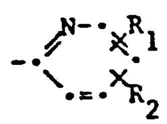

et

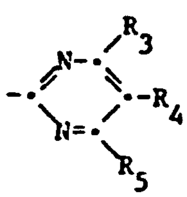

$R_1$ à $R_5$ représentent chacun, indépendamment les uns des autres, l'hydrogène, un méthyle ou un éthyle, $X_1$ represente l'hydrogène, $X_2$ représente l'hydrogène, le chlore ou un méthyle et $X_3$ l'hydrogène, un méthyle, le chlore, -CN, -COOH, -$CONH_2$, un phényle, un méthylphényle, un méthoxyphényle, un cyclohexyle, un pyridyle, un imidazolyle, un pyrrolidinyle, un -COO-Alkyl contenant de 1 à 12 atomes de carbone dans sa partie alkyle, un-COO-phényl,

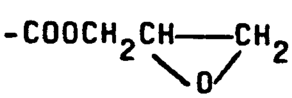

un -COO-Alkyl-OH dont l'alkyle contient de 1 à 4 atomes de

carbone, un -OCO-Alkyl contenant de 1 à 4 atomes de carbone dans sa partie alkyle, un -OCO-Phényl, un -CO-Alkyl dont l'alkyle contient de 1 à 3 atomes de carbone, un alcoxy en $C_1$-$C_{20}$ ou un phénoxy, ou $X_2$ représente l'hydrogène tandis que $X_1$ et $X_3$ forment ensemble un radical d'anhydride ou un radical -CO-NR'''-CO- ou représentent chacun un -COOH ou un -COO-Alkyl contenant de 1 à 6 atomes de carbone dans sa partie alkyle, le symbole R''' représentant un alkyle en $C_1$-$C_{18}$ linéaire ou ramifié, un cyclohexyle ou un phényle éventuellement porteur d'un ou de deux substituants pris dans l'ensemble constitué par les alkyles en $C_1$-$C_6$, les halogènes, le radical cyano, le radical nitro et les radicaux alcoxy en $C_1$-$C_3$.

9.- Polymères linéaires de masse moléculaire moyenne comprise entre 2000 et 1 500 000 et/ou leurs complexes selon la revendication 8, qui sont constitués de motifs de formule 8 et/ou de complexes, conformes à la définition, de motifs (8) de ce genre, dans lesquels X a la signification donnée à la revendication 8, $R_1$, $R_2$ et $R_4$ représentent chacun l'hydrogène et $R_3$ et $R_5$ chacun un methyle, ou $R_1$ à $R_5$ représentent chacun l'hydrogène, de 5 à 100 % des motifs de formule 8 étant complexés par des métaux ou des composés de métaux qui ne sont ni des métaux alcalins, ni des métaux alcalinoterreux, ni des composés de métaux alcalins, ni des composés de métaux alcalinoterreux.

10.- Complexes de polymères selon l'une des revendications 7 et 8, dans lesquels le métal est un métal des groupes secondaires Ib et VIII, en particulier le fer, le ruthénium, le palladium, le rhodium, le cobalt, le nickel le platine ou le cuivre.

11.- Complexes de polymères selon l'une des revendications 7 et 8, dans lesquels le métal est le ruthenium, le palladium, le cobalt, le platine ou le cuivre.

12.- Application de polymères complexés, au moins partiellement, par du palladium ou un composé du palladium, selon l'une des revendications 7 et 8, comme catalyseurs pour des réactions de transvinylation.